# EUROPEAN PATENT SPECIFICATION

(11) **EP 3 981 432 B1**
(45) Date of publication and mention of the grant of the patent: **31.07.2024**
(21) Application number: 20779050.2
(22) Date of filing: 27.03.2020
(51) Int. Cl.: A61K 31/436, A61K 45/00, A61P 3/10, A61P 25/02, A61K 38/13, A61K 31/706

(54) **THERAPEUTIC AGENT FOR WOLFRAM SYNDROME**
THERAPEUTISCHES MITTEL FÜR DAS WOLFRAM-SYNDROM
AGENT THÉRAPEUTIQUE POUR LE SYNDROME DE WOLFRAM

(30) Priority: 27.03.2019 JP 2019061768
(43) Date of publication of application: 13.04.2022
(73) Proprietor: TOKYO MEDICAL UNIVERSITY, Shinjyuku-ku Tokyo 160-8402 (JP); AYUMI Pharmaceutical Corporation, Tokyo 104-0061 (JP)
(72) Inventor: NAKAJIMA Toshihiro, Yokohama-shi, Kanagawa 225-0023 (JP); FUJITA Hidetoshi, Chofu-shi, Tokyo 182-0006 (JP); KURODA Masahiko, Tokyo 167-0051 (JP); KANEKURA Kohsuke, Tokyo 167-0051 (JP); HIRAMOTO Masaki, Tokyo 160-0022 (JP); KUMAGAI Katsuyoshi, Saitama-shi, Saitama 337-0006 (JP); AONO Hiroyuki, Osaka-shi, Osaka 541-0044 (JP)
(74) Representative: Moser Götze & Partner Patentanwälte mbB
(86) International application number: PCT/JP2020/014194
(87) International publication number: WO 2020/196873

(56) References cited:
- JP-A- 2014 531 425
- US-A1- 2015 246 037
- US-A1- 2015 246 037
- TAKASHI HARA ET AL: "Calcium Efflux From the Endoplasmic Reticulum Leads to ?-Cell Death", ENDOCRINOLOGY, vol. 155, no. 3, 1 March 2014 (2014-03-01), US, pages 758 - 768, XP055745058, ISSN: 0013-7227, DOI: 10.1210/en.2013-1519
- MEUNIER I ET AL: "[Hereditary optic neuropathies: from clinical signs to diagnosis].", JOURNAL FRANCAIS D'OPHTALMOLOGIE DEC 2013, vol. 36, no. 10, December 2013 (2013-12-01), pages 886 - 900, XP009540311, ISSN: 1773-0597
- TAKASHI HARA, JANA MAHADEVAN, KOHSUKE KANEKURA, MARIKO HARA, SIMIN LU, FUMIHIKO URANO: "Calcium Efflux From the Endoplasmic Reticulum Leads to beta- Cell Death", ENDOCRINOLOGY, vol. 155, no. 3, 2014, pages 758 - 768, XP055745058
- CHIH-HAO WANG, YI-FAN CHEN, CHIA-YU WU, TING-FEN TSAI, YAU-HUEI WEI: "Cisd2 deficiency leads to impaired white adipogenesis and insulin insensitivity in mice", BIOCHIMICA ET BIOPHYSICA ACTA - BIOENERGETICS, vol. 1817, no. 1, 1 October 2012 (2012-10-01), NL, pages S88, XP055860660, ISSN: 0005-2728, DOI: 10.1016/j.bbabio.2012.06.240
- MARIA TERESA PALLOTTA, GIORGIA TASCINI , ROBERTA CRISPOLDI , CIRIANA ORABONA , GIADA MONDANELLI , URSULA GROHMANN AND SUSANNA ESP: "Wolfram syndrome, a rare neurodegenerative disease: From pathogenesis to future treatment perspectives", JOURNAL OF TRANSLATIONAL MEDICINE, vol. 17, no. 1, July 2019 (2019-07-01), pages 1 - 12, XP055745064
- N. B. TOPPINGS, J. M. MCMILLAN, P. Y. B. AU, O. SUCHOWERSKY, L. E. DONOVAN: "Wolfram Syndrome: A Case Report and Review of Clinical Manifestations, Genetics Pathophysiology, and Potential Therapies", CASE REPORTS IN ENDOCRINOLOGY, vol. 2018, 2018, pages 1 - 8, XP055745067
- SAKUTA, HIDENARI; ITO, TOSHIMITSU: "Endoplasmic reticulum (ER) stress and obesity/diabetes: Overnutrition impairs pancreatic beta cells", NATIONAL DEFENSE MEDICAL JOURNAL, vol. 62, no. 11.12, 1 November 2015 (2015-11-01), JP, pages 149 - 159, XP009531292, ISSN: 0006-5528

## Description

### TECHNICAL FIELD

The present invention relates to a therapeutic agent for Wolfram syndrome.

### BACKGROUND ART

Japanese Patent No. 5221392 disclose a therapeutic agent for Wolfram syndrome containing a 3,5-seco-4-nor-cholestane derivative as an active ingredient. The articles "Calcium Efflux From the Endoplasmic Reticulum Leads to Cell Death" by Takashi Hara ET AL, the patent application US 2015/246037 A1 as well as the article "Hereditary optic neuropathies: from clinical signs to diagnosis" by MEUNIER I ETAL, disclose the treatment of Wolfram syndrome.

Patent Document 1: Japanese Patent No. 5221392

### DISCLOSURE OF THE INVENTION

The scope of the present invention is defined in the claims; any other disclosure in the present description not protected by the appended claims, regardless of whether it is indicated as being preferred or exemplified, is provided for information purposes, only.

### PROBLEMS TO BE SOLVED BY THE INVENTION

The present invention has an object to provide a new therapeutic agent for Wolfram syndrome.

### SOLUTIONS TO THE PROBLEMS

The present invention is based on the following knowledge.

Wolfram syndrome presents central nervous system manifestations such as type I diabetes, diabetes insipidus, hearing loss, sight loss, and convulsion from a young age, and causes deaths before the age of 30. It is a rare intractable disease in which estimated presence of patients is 150-200 in Japan and 30,000 worldwide. Its causative gene (WFS1) is considered to function mediated by the stability of synoviolin. In other words, it is considered that synoviolin becomes unstable in a patient having a mutant WFS1 and various kinds of states of disease are present. As indicated by the embodiments, synoviolin is responsible for regulation of insulin secretion mediated by a transcriptional coactivator CREB regulated transcription coactivator 2 (CRTC2) in pancreatic β cells. Since nuclear translocation of the CRTC2 is controlled by calcineurin, when ciclosporin A (CsA) as a calcineurin inhibitor was administered to a pancreatic β cell specific synoviolin knockout mouse with type I diabetes, the high blood glucose was normalized. Consequently, the calcineurin inhibitor is considered to function as a therapeutic agent for Wolfram syndrome.

The first aspect disclosed in this description relates to a therapeutic agent for Wolfram syndrome containing a calcineurin inhibitor as an active ingredient.

A preferred example of the calcineurin inhibitor includes ciclosporin, a pharmaceutically acceptable salt of ciclosporin, or a pharmaceutically acceptable solvate of ciclosporin, tacrolimus, a pharmaceutically acceptable salt of tacrolimus, or a pharmaceutically acceptable solvate of tacrolimus.

A preferred example of the calcineurin inhibitor other than the above includes rapamycin, a pharmaceutically acceptable salt of rapamycin, or a pharmaceutically acceptable solvate of rapamycin.

### ADVANTAGEOUS EFFECTS OF THE INVENTION

According to the present invention, a therapeutic agent for Wolfram syndrome can be provided.

### BRIEF DESCRIPTION OF THE DRAWINGS

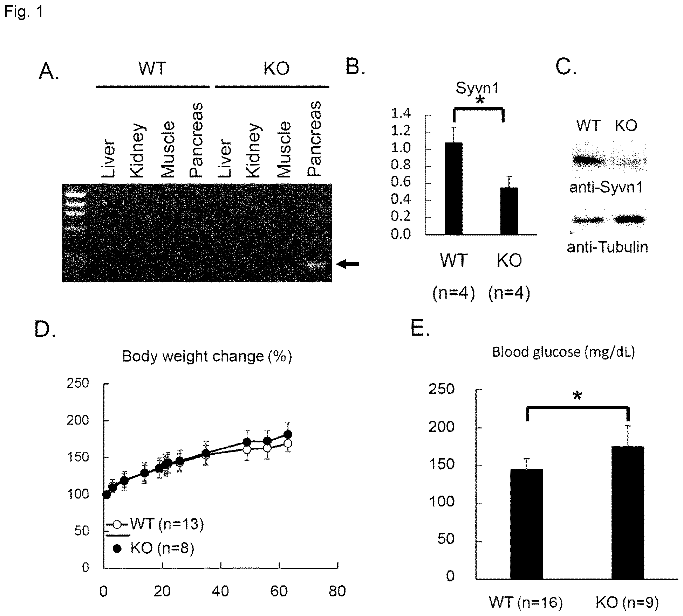

Fig. 1A is a gel electrophoresis photograph illustrating organ specificity in gene disruption for respective organs (liver, kidney, muscle, and pancreas) of a wild type (WT) mouse and a Syvn1 knockout (KO). In the KO, an abnormal exon destroyed by a Cre-loxP system appears (arrow). Fig. 1B is a graph illustrating Syvn1 mRNA expression levels in pancreas of the wild type (WT) mouse and the Syvn1 knockout (KO). Fig. 1C is a photograph illustrating Syvn1 protein expression levels in pancreas of the wild type (WT) mouse and the Syvn1 knockout (KO). Fig. 1D illustrates variations of weights of the wild type (WT) mouse and the Syvn1 knockout (KO). The horizontal axis indicates a day, and the vertical axis indicates the weight variation with the day at 0 as 100%. Fig. 1E is a graph illustrating blood glucose levels of the wild type (WT) mouse and the Syvn1 knockout (KO).

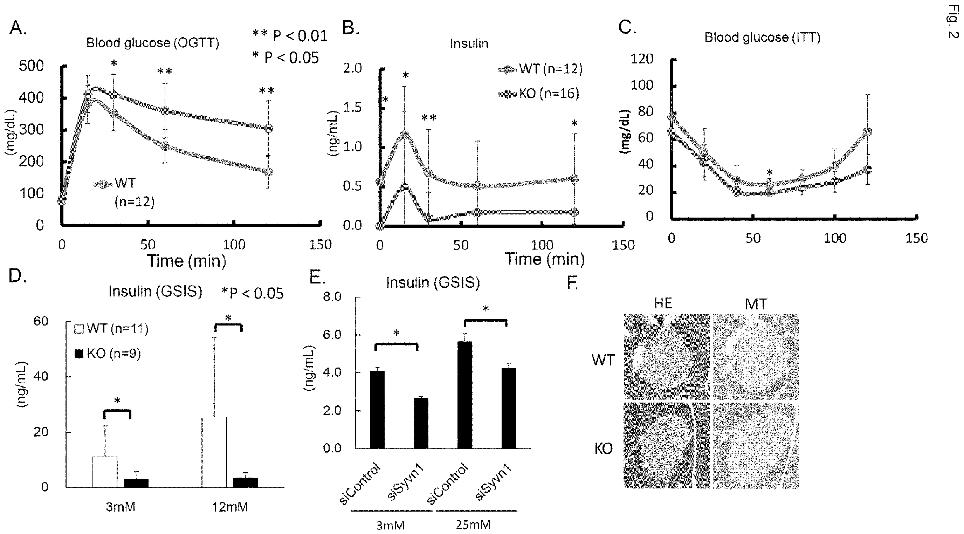

Fig. 2A is a graph illustrating variations of the blood glucose levels by glucose loading. Fig. 2B is a graph illustrating variations of blood insulin concentration in the glucose loaded mice. Fig. 2C is a graph illustrating a result of an insulin resistance test. The horizontal axis indicates a time period (minute), and the vertical axis indicates the blood glucose level. Fig. 2D is a graph illustrating an examination result of a glucose responsive insulin secretory reserve in pancreatic islets of the wild type (WT) mouse and the Syvn1 knockout (KO). Fig. 2E is a graph illustrating a measurement result of an amount of insulin secretion by glucose stimulation. Fig. 2F is a photograph of stained tissues indicating results of a histochemical examination of pancreas of the wild type (WT) mouse and the Syvn1 knockout (KO).

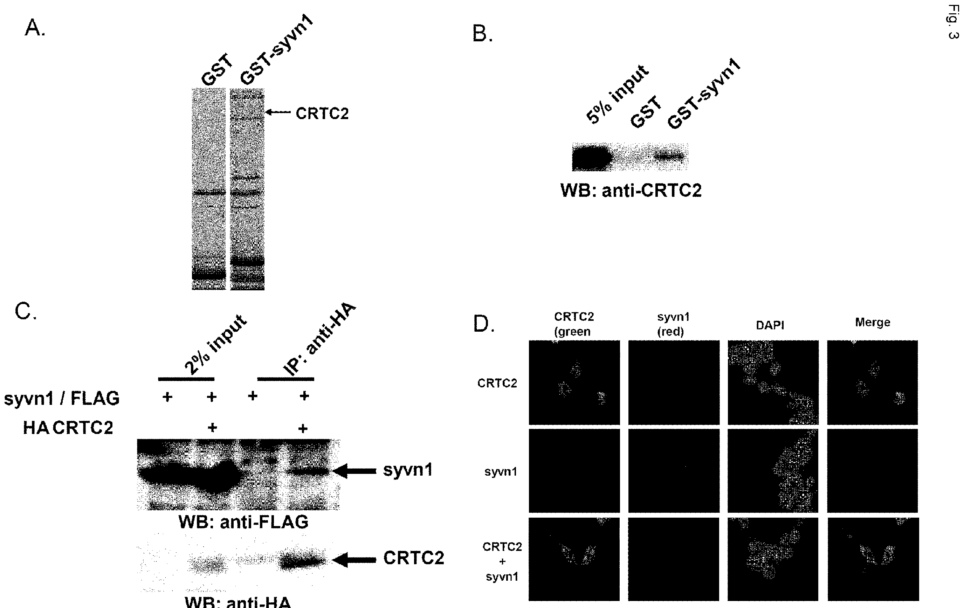

Fig. 3A is a Western blot illustrating binding of CTRC2 as a binding molecule of Syvn1 with synoviolin. Fig. 3B is a photograph illustrating binding of GST fusion Syvn1 with CRTC2. Fig. 3C is a photograph illustrating binding of Syvn1 with CRTC2 by immunoprecipitation. Fig. 3D is a photograph illustrating colocalization of Syvn1 and CRTC2.

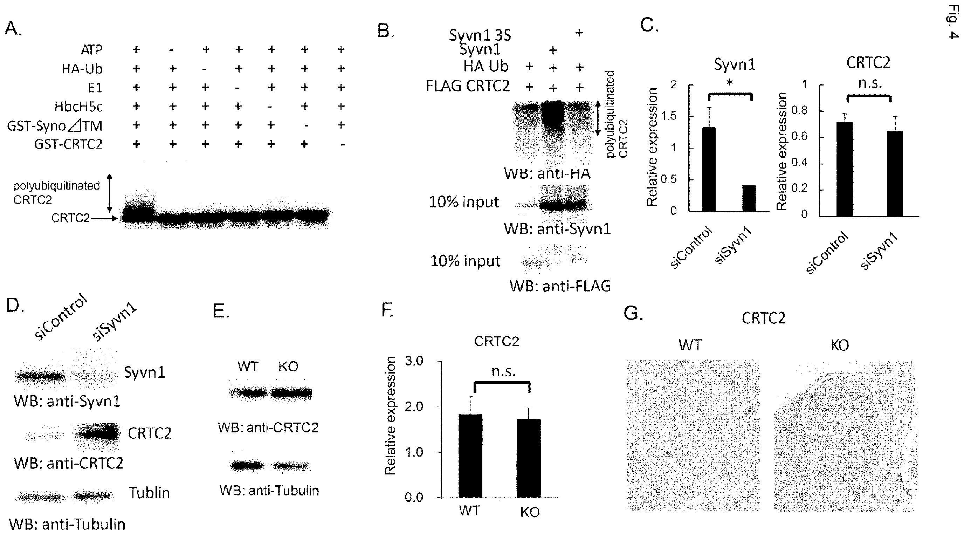

Fig. 4A is a Western blot illustrating ubiquitination of CRTC2 by synoviolin in vitro. Fig. 4B is a Western blot illustrating ubiquitination of CRTC2 by synoviolin and its mutant. Fig. 4C is a graph illustrating levels of CTRC2 mRNA expression by siSyvn1. Fig. 4D is a Western blot illustrating levels of CTRC2 expression by siSyvn1. Fig. 4E is a Western blot illustrating the CTRC2 expression levels in pancreatic islets of the KO and the WT mouse. Fig. 4F is a graph illustrating CTRC2 mRNA in the pancreatic islets of the KO and the WT mouse. Fig. 4G is a picture which relates to CTRC2 mRNA in the pancreatic islets of the KO and the WT mouse.

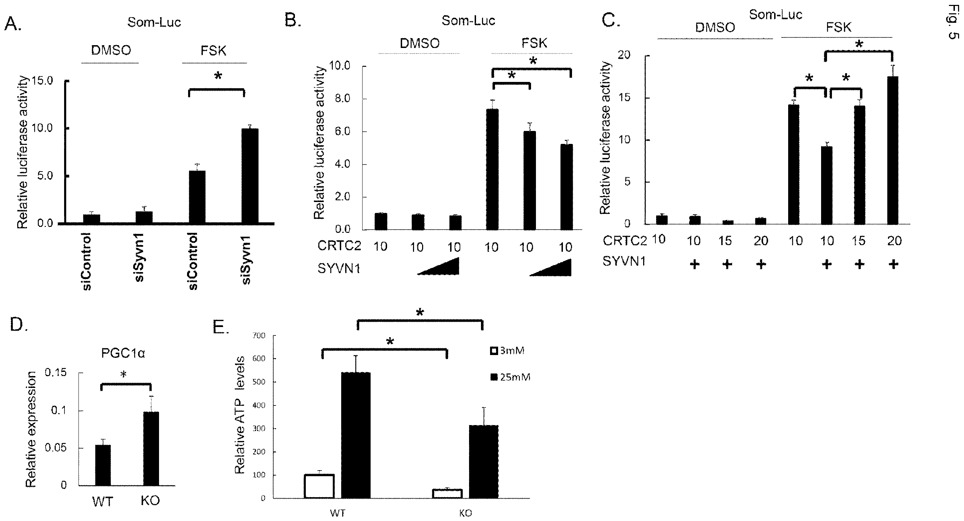

Fig. 5A is a graph illustrating influences of synoviolin inhibition on FSK-stimulated transactivation. Fig. 5B is a graph illustrating influences of CTRC2 and synoviolin on the FSK-stimulated transactivation. Fig. 5C is a graph illustrating a dose dependency of CTRC2 relative to the FSK-stimulated transactivation. Fig. 5D is a graph illustrating PGC1α expression levels in the pancreatic islets of the KO and the WT mouse. Fig. 5E is a graph illustrating ATP levels in the pancreatic islets of the KO and the WT mouse.

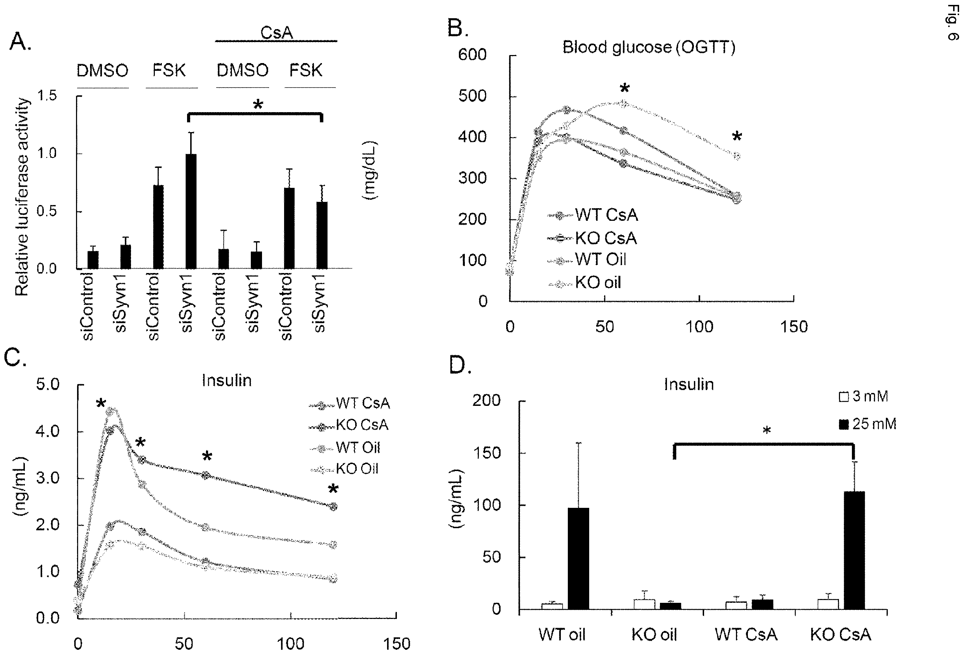

Fig. 6A is a graph illustrating a CsA activation action to the synoviolin inhibition relative to the FSK-stimulated transactivation. Fig. 6B is a graph illustrating an effect of CsA in accordance with increase in blood glucose level caused by glucose loading in the KO and the WT mouse. Fig. 6C is a graph illustrating an effect of CsA relative to the change of insulin amount in the KO and the WT mouse. Fig. 6D is a graph illustrating an effect of CsA relative to the change of insulin amount by glucose stimulation in the pancreatic islets of the KO and the WT mouse.

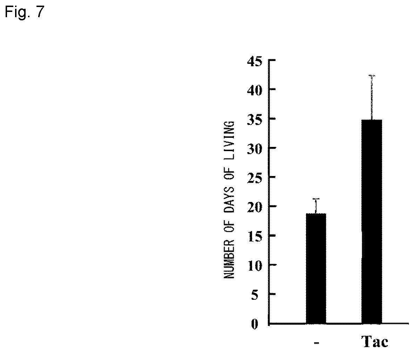

Fig. 7 is a graph illustrating a life-prolonging effect of tacrolimus to death from neurological disorders of a nerve specific Syvn1 deficient mouse (Syn-Cre, Syvn1^{fl/fl}).

### DESCRIPTION OF PREFERRED EMBODIMENTS

The first aspect disclosed in this description relates to a therapeutic agent for Wolfram (Wolfram) syndrome that contains a calcineurin (Calcineurin) inhibitor as an active ingredient by an effective dose. Preferred examples of the calcineurin inhibitor include ciclosporin, a pharmaceutically acceptable salt thereof or a pharmaceutically acceptable solvate thereof, tacrolimus, and a pharmaceutically acceptable salt thereof or a pharmaceutically acceptable solvate thereof.

The pharmaceutically acceptable salts of various kinds of calcineurin (Calcineurin) inhibitors and the pharmaceutically acceptable solvates of various kinds of calcineurin (Calcineurin) inhibitors are also effective as active ingredients of the therapeutic agents for Wolfram (Wolfram) syndrome. The exemplary pharmaceutically acceptable salt is a known salt such as a hydrochloride salt and a sodium salt. The exemplary pharmaceutically acceptable solvate is a hydrate.

Calcineurin is a calcium/calmodulin regulatory protein phosphatase involved in intracellular signal transduction. The calcineurin inhibitor is a substance, for example, that targets a calcineurin phosphatase (PP2B, PP3) as a cellular enzyme involved in a gene regulation to block a calcineurin dephosphorylation of a suitable substrate.

The calcineurin inhibitor is preferably an immunophilin-binding compound having a calcineurin suppressive activity. An immunophilin-binding calcineurin inhibitor is a compound that forms a calcineurin inhibiting complex with immunophilin, for example, cyclophilin and macrophyllin. The cyclophilin-binding calcineurin inhibitor is exemplified by ciclosporin or a ciclosporin derivative (hereinafter, ciclosporin), and the macrophyllin-binding calcineurin inhibitor is exemplified by ascomycin (FR 520) and an ascomycin derivative (hereinafter, ascomycin). A wide range of ascomycin derivatives that are native or obtained by manipulating a fermentation method or chemical derivatization among fungal species have been known. An ascomycin type macrolide includes ascomycin, tacrolimus (FK506), sirolimus, and pimecrolimus.

Cyclosporin (ciclosporin) extracted at first from Potypaciadium inifilatum as a soil fungal species has a cyclic 11-amino acid structure, and includes, for example, ciclosporin A to I, for example, ciclosporin A, B, C, D, and G. Ciclosporin binds with cytoplasmic protein cyclophilin of an immune responsive lymphocyte, especially a T lymphocyte, thus forming a complex. The complex inhibits calcineurin that induces transcription of interleukin-2 (IL-2) under an ordinary environment. Ciclosporin also inhibits lymphokine production, releases interleukin, and reduces a function of an effector T cell.

Voclosporin is a calcineurin inhibitor of a next generation that is a semisynthetic derivative of ciclosporin A and is more powerful and low in toxicity. Like other molecules in this class, voclosporin reversibly inhibits the immune responsive lymphocyte, especially the T lymphocyte, and inhibits the production and release of lymphokine. This action is mediated by the calcineurin inhibition at first, and the phosphatase enzyme was found in cytoplasm of a cell. Voclosporin has a single carbon extension provided by a double bond found with a deeper extension in a latch/regulatory region of calcineurin. In the embodiment, the composition in the content of this disclosure is voclosporin having a trans form of trans-ISA247 CAS RN 368455-04-3, and this is disclosed in, for example, U.S. Patent Publication No. 2006/0217309 Furthermore, the voclosporin composition is disclosed in, for example, U.S. Patent No. 7, 060, 672.

Tacrolimus (FK506) is a fungal product, while it is also another calcineurin inhibitor having a macrolide lactone structure. Tacrolimus is used as an immunosuppressive agent along with a transplantation of liver, kidney, heart, lung, and heart/lung. It has been found that tacrolimus inhibits IL-2 production. Tacrolimus binds with immunophilin (FK-binding protein 12, FKBP12), and subsequently, the complex binds with calcineurin, thereby inhibiting its phosphatase activation.

Sirolimus (rapamycin) is a product of a microorganism isolated from Streptomyces hygroscopicus as an actinomycetes. Sirolimus binds with immunophilin (FK-binding protein 12, FKBP12) that forms a complex directly binding with an mTOR complex 1 (mTORC1) to inhibit a mammal target of rapamycin (mTOR) pathway. Sirolimus inhibits a response to interleukin-2 (IL-2), thereby inhibiting activation of T and B cells. In contrast, tacrolimus and ciclosporin inhibit IL-2 production.

Pimecrolimus is, similarly to tacrolimus, a new calcineurin inhibitor found with an antifungal property to Malassezia spp.

An agent of one aspect disclosed in this description can be used by both oral and parenteral administration. In the case of the parenteral administration, a pulmonary preparation (for example, one using nebulizer), a nasal preparation, a transdermal preparation (for example, ointment and cream formulation), an injection preparation, and the like are included. The injection preparation can be systemically or locally administered by an intravenous injection such as a drip infusion, an intramuscular injection, an intraperitoneal injection, a subcutaneous injection, and the like.

The administration method is appropriately selected based on the age and the symptom of the patient. An effective dose is 0.1 µg to 100 mg, and preferably 1 to 10 µg per kg of weight at a time. However, the dose of the above-described therapeutic agent is not limited thereto. When a nucleic acid such as siRNA is mixed, an exemplary amount of the nucleic acid is 0.01 to 10 µg/ml, and preferably 0.1 to 1 µg/ml.

An agent of one aspect disclosed in this description can be formulated according to a usual method, and may contain pharmaceutically acceptable carrier and additive. As such carrier and additive, water, a pharmaceutically acceptable organic solvent, collagen, polyvinyl alcohol, polyvinylpyrrolidone, carboxyvinyl polymer, carboxymethylcellulose sodium, sodium polyacrylate, sodium alginate, water-soluble dextran, sodium carboxymethyl starch, pectin, methyl cellulose, ethyl cellulose, xanthan gum, gum arabic, casein, agar, polyethylene glycol, diglycerin, glycerin, propylene glycol, vaseline, paraffin, stearyl alcohol, stearic acid, human serum albumin, mannitol, sorbitol, lactose, a surfactant acceptable as a pharmaceutical additive, and the like are included.

The above-described additives are selected alone or in combination as necessary from the above depending on the agent type of the therapeutic agent of the present invention. For example, in the case of using as an injection preparation, one in which a calcineurin inhibitor is dissolved in a solvent (for example, a normal saline, a buffer solution, a glucose solution) and Tween 80, Tween 20, gelatin, human serum albumin, or the like are added thereto can be used. Alternatively, one freeze-dried to obtain an agent type to be dissolved before use may be employed. As an excipient for freeze-drying, for example, sugar alcohols and sugars such as mannitol and glucose are usable.

This description discloses a use of the calcineurin inhibitor for manufacturing an agent for treating Wolfram syndrome (therapeutic agent for Wolfram syndrome). This use is a method for manufacturing a therapeutic agent for Wolfram syndrome using a calcineurin inhibitor as an active ingredient by a known preparation method and a known processing method.

This description discloses a method for treating Wolfram syndrome of a target mammal including a step of administrating a therapeutic agent for Wolfram syndrome to the target mammal (human or non-human mammal). The target takes the therapeutic agent for Wolfram syndrome containing the therapeutic agent for Wolfram syndrome as an active ingredient, thereby providing an expectation for the treatment of Wolfram syndrome.

A knockout animal such as a pancreatic β cell specific knockout mouse can be considered as a model animal of Wolfram syndrome. Therefore, this description also provides a model animal of Wolfram syndrome in which synoviolin is knocked out.

### [Example 1]

### Material and Method

### Mouse

An animal experiment was conducted according to in-facility and domestic guidelines for animal experiments, and it was approved in advance by the in-facility animal management and use committee of Tokyo Medical University. The mice were bred and subjected to the experiment under a condition of a light-dark cycle of 12 hours (temperature 20°C to 26°C; humidity 40% to 65%) in an SPF environment. F-1 (5.1% fat, 21.3% protein: Funabashi Farm, Chiba, Japan) was given as a feed and a tap water was given as a drinking water. All the mice used for the study were C57BU6J strain, and for producing a mouse whose pancreatic β cell Syvn1 was specifically knocked out, a floxed homozygous Syvn1^{flox/flox} mouse was crossed with a heterozygous Pdx1-Cre mouse (Jackson Laboratories) to produce a double heterozygous Pdx1-Cre; homozygous Syvn1^{flox/+} mouse, and this mouse was crossed with a homozygote Syvn1^{flox/flox} mouse, thereby producing a Syvn1 homozygote (Pdx1-Cre; ^{flox/flox}), and a homozygote Syvn1^{flox/flox}.

### Glucose Tolerance Test

After the mice were fasted for 16 hours, the mice were provided with 1.5 mg/g of glucose by oral administration. Blood samples were successively extracted from tails, and the blood glucose levels were measured using an automatic blood glucose meter (Glutest Neo alpha; Sanwa Chemical, Nagoya, Japan). Insulin concentrations were measured according to a protocol of ELISA kit (FUJIFILM Wako Shibayagi, Gunma, Japan).

### Insulin Resistance Test

After the mice were fasted for 16 hours, 0.75 mU/g of human derived insulin (Novolin R; Novo Nordisk, Denmark) was intraperitoneally administered to the mice. Blood samples were successively extracted from tails, and the insulin concentrations were measured using the ELISA kit.

### Test Results and Considerations

Examination results on phenotype of the pancreatic β cell specific Syvn1 knockout (KO) mouse are illustrated in Fig. 1. Fig. 1A is a gel electrophoresis photograph illustrating organ specificity in gene disruption for respective organs (liver, kidney, muscle, and pancreas) of a wild type (WT) mouse and a Syvn1 knockout (KO). In the KO, an abnormal exon destroyed by a Cre-loxP system appears (arrow). Fig. 1B is a graph illustrating Syvn1 mRNA expression levels in pancreas of the wild type (WT) mouse and the Syvn1 knockout (KO). Fig. 1C is a photograph illustrating Syvn1 protein expression levels in pancreas of the wild type (WT) mouse and the Syvn1 knockout (KO). Fig. 1D illustrates variations of weights of the wild type (WT) mouse and the Syvn1 knockout (KO). The horizontal axis indicates a day, and the vertical axis indicates the weight variation with the day at 0 as 100%. Fig. 1E is a graph illustrating blood glucose levels of the wild type (WT) mouse and the Syvn1 knockout (KO). The synoviolin expression level in pancreas was decreased (Fig. 1B and Fig. 1C) and the blood glucose level was statistically significantly high (Fig. 1E) in the Syvn1 knockout (KO) compared with the wild type (WT). On the other hand, the insulin secretory reserve having an important role on maintaining a normal glucose concentration in serum was not affected.

Furthermore, for examining glucose responsive blood glucose level regulation and insulin secretion, glucose was orally administered to the wild type (WT) and the Syvn1 knockout (KO) mouse. Variations of the blood glucose levels by glucose loading are illustrated in Fig. 2A. Consequently, a glucose clearance was statistically significantly low in the KO mouse compared with the WT. Furthermore, Fig. 2B illustrates variations of blood insulin concentration in the glucose loaded mice. While the increase of the insulin concentration in accordance with the increase of the blood glucose was recognized in the WT, the increase of the blood insulin concentration was significantly low in the KO mouse compared with the WT.

Next, an insulin resistance test was conducted to examine an insulin responsiveness of the mice, and the results are illustrated in Fig. 2C, Fig. 2D. While the blood glucose was decreased by the insulin administration in the WT and the KO mouse, there was no difference between both mice. In a histochemical examination, differences in tissue were not recognized (Fig. 2F).

Consequently, since reduction in insulin secretion function was recognized in the KO mouse in which the pancreatic β cell specific synoviolin was knocked out, and this is remarkably similar to a state of disease of a Wolfram syndrome patient, for example, an increase of blood glucose level caused by unstable synoviolin, this model can be considered as one animal model of Wolfram syndrome.

### Pancreatic Islets Isolation and β Cell Preparation

The pancreatic islets were isolated from the KO and the WT mice. The common bile duct was clamped at a point near the duodenal outlet, and then, HBSS buffer (Sigma) containing HEPES (Sigma) and Liberase (Roche Diagnostics, Mannheim, Germany) was injected into the common bile duct. The enlarged pancreas was extracted and incubated at 37°C for 24 minutes. The pancreas was dispersed by pipetting, and washed twice by the HBSS buffer containing 10% of FBS. The pancreatic islets were collected by manual picking. For analyzing the insulin secretion, the insulin secretion from the isolated pancreatic islets was measured using a Krebs-Ringer bicarbonate buffer solution having a glucose concentration of 2.8 mmol/L. After a pre-incubation with an ordinary glucose concentration for 30 minutes, an incubation was performed at 37°C for one hour using ten pancreatic islets per culture container. The insulin level was determined using the ELISA kit according to a protocol (FUJIFILM Wako Shibayagi, Gunma, Japan).

### Cell Culture and Transient Transfection Assay

Seeding was performed on a plate so as to have 5×10⁶ pieces of HEK293 and HEK293T cells, and the cells were cultured on Dulbecco's Modified Eagle's Medium according to a usual method. Rat INS cells were provided from Dr. Claes B. Wollheim, and cultured at 1 to 5×10⁶ pieces using RPMI 1640 culture fluid. The transient transfection was performed using Lipofectamine 2000 according to a protocol (Invitrogen). 24 hours after the transfection, the cells were dissolved by a cell lysis buffer (Promega, Madison, Wl, USA), and a luciferase activity was measured. For ensuring an equivalent amount of DNA, an empty plasmid was added to each transfection.

Using the rat INS cell as a cultured cell derived from the β cell, the amount of insulin secretion by the cell introduction in the synoviolin specific siRNA was measured.

### Plasmid And Antibody

A full-length CRTC2 sequence was PCR-amplified from a mouse cDNA. Fragments of the CRTC2 deficient mutant was obtained by the PCR amplification, and they were inserted into a pcDNA3 HA plasmid or a pcDNA3 FLAG plasmid (Invitrogen, Carlsbad, CA, USA) with the full-length CRTC2. A plasmid sequence was confirmed by a sequence determination. As the SYVN1 plasmid, previously described one was used. Antibodies below were used: Anti-FLAG (M2) (Sigma), Anti-Tubulin (Sigma), Anti-HA (12CA5 and 3F10; Roche, Indianapolis, IN, the United States of America), Anti-TORC2 (Novus Biologicals, CO, the United States of America, and Proteintech, IL, the United States of America). As an anti-SYVN1 rabbit polyclonal antibody, previously described one was used.

### GST Pull Down Assay

A GST fusion protein was expressed and purified using glutathione sepharose beads (GE Healthcare, Little Chalfont, UK). A cell extract was incubated at 4°C for four hours with each GST fusion protein binding to sepharose beads by 1 ml of a buffer solution A [20 mM tris-HCl, 100 mM NaCl, 1 mM ethylenediaminetetraacetic acid (EDTA), 1 mM dithiothreitol (DTT), 0.1% Nonidet P-40 (NP-40), 5% glycerol, 1 mM Na₃VO₄, 5 mM NaF, 1 µg/ml aprotinin, 1 µg/ml leupeptin, pH 8.0]. After washing with the buffer solution A, the binding protein was fractionated by a sodium dodecyl sulfate polyacrylamide gel electrophoresis (SDS-PAGE), and subsequently, detected by Western blotting.

### Immunoprecipitation Assay

HA-CRTC2 and SYVN1/FLAG expression vectors were transfected into HEK293T cells. After 24 hours, the cells were dissolved in 200 µL of a lysis buffer (20 mM Tris-HCl, pH 8.0; 100 mM NaCl; 1 mM EDTA; 1 mM DTT; 1% NP-40; 5% glycerol; and protease inhibitor). The dissolved product was diluted in a binding buffer (composition the same as that of the lysis buffer except that 0.1% of NP-40 was contained), and mixed with 20 µL of an anti-HA antibody binding to the protein G-sepharose beads. After the incubation at 4°C for four hours, the beads were washed three times by the binding buffer. The binding protein was fractionated by SDS-PAGE, and analyzed by Western blotting. For detecting an interaction between endogenous SYVN1 and CRTC2, HEK293 cells were dissolved in a solution containing 100 mM tris-HCl, 80 mM NaCl, 1 mM EDTA, 5 mM ethylene glycol tetraacetic acid, 5% glycerol, 2% (w/v) digitonin, 0.1% Brij 35, protease inhibitor cocktail, and 20 mM MG132. Immunoprecipitation was performed using an anti-CRTC2 antibody or a control IgG, and then, Synv1 was detected using an anti-Syvn1 antibody by Western blotting.

### In Vitro Ubiquitination Assay

In vitro ubiquitination assay was performed according to a usual method. Briefly describing, GST-CRTC2 was incubated at 37°C for two hours with 0.75 µg of HA-Ub, 125 ng of E1 (Biomol International, Pennsylvania in the United States of America, Plymouth Meeting), 150 ng of UBcH5C, and 150 ng of maltose-binding protein tagged SYVN1ΔTM-His (50 µl), and a reaction solution containing 1 mM tris-HCl, pH7.5; 5 mM MgCl₂; 0.6 mM DTT; and 2 mM ATP. Subsequently, glutathione Sepharose was added, and the mixture was washed by a GST wash buffer (50 mM Tris-HCl, pH 7.5; 0.5 M NaCl; 1% Triton X; 1 mM EDTA; 1 mM DTT; and protease inhibitor). The ubiquitinated CRTC2 was analyzed by Western blotting using an anti-HA antibody.

### In Vivo Ubiquitination Assay

Seeding was performed on a plate so as to have 5×10⁶ pieces of HEK293T cells, and transfection was performed with HA-CRTC2, Ub/FLAG, SYVN1, or SYVN1 3S expression plasmid. After 24 hours, 20 µM of MG-132 was added, and the cells were dissolved in a lysis buffer (50 mM of HEPES, pH 7.9; 150 mM of KCI; 1 mM of phenylmethanesulfonyl fluoride, 1% of Triton X-100; 10% of glycerol; and protease inhibitor). The dissolved product was mixed with 1 µg of the anti-HA antibody conjugated to protein G-sepharose beads. After the incubation at 4°C for four hours, the beads were washed three times by the binding buffer. The binding protein was fractionated by SDS-PAGE, and analyzed by an immunoblotting.

### Immunocytochemistry

An immunocytochemistry inspection was performed according to a usual method. Briefly describing, a plasmid was transfected into the cell, it was fixed with 3.7% formaldehyde after 24 hours from the transfection, a transmission process was performed with 0.2% triton X-100, and it was blocked with 2% bovine serum albumin. The cells were incubated with a primary antibody of a rat anti-HA (1:1,000) or a rabbit anti-FLAG (1:1,000), and subsequently, stained by Alexa Fluor 594 anti-mouse antibody (Molecular Probes, Eugene, OR, USA) or Alexa Fluor 488 Anti-rat secondary antibody (1:1,000) (Molecular Probes, Eugene, OR, USA).

### RNA Interference and Real Time PCR

The siRNA for human, mouse, and rat SYVN1 was purchased from Ambion Inc. (Austin, Texas, the United States of America). After the siRNA was dissolved in a DNase/RNase free water, siRNA transfection was performed using Lipofectamine 2000. Total RNA from the pancreatic islets was purified according to a protocol of RNeasy (Qiagen), and reverse-transcribed by a random primer using ReverTra Ace (Toyobo, Osaka City, Japan). Real time PCR was performed using LightCycler 480 Probes Master (Roche Diagnostics, Mannheim, Germany). The expression level was determined in a comparison with that of 18s rRNA.

### Results

For directly evaluating the function in the pancreatic islets, a glucose responsive insulin secretory reserve in the pancreatic islets of the WT or KO mouse was examined. The results were illustrated in Fig. 2D. While the amount of insulin secretion was increased depending on the glucose concentration in the WT mouse, the increase of insulin secretion caused by the glucose stimulation was not recognized in the β cells of the KO.

Furthermore, the synoviolin specific siRNA (siSyvn1) was introduced into cells using rat INS cells, and the synoviolin function was knocked down, thus resulting in significantly suppressing the amount of insulin secretion caused by the glucose stimulation compared with a control siRNA (siControl) (Fig. 2E). Consequently, it was suggested that the reduction of the synoviolin function in the β cells inhibited the insulin secretion and caused a symptom of high blood glucose or the like.

Next, a factor binding to synoviolin in the pancreatic β cells were searched. In an examination using cultured cells (HEK293 cells) from kidney and INS cells from β cells, it was indicated that synoviolin was bound to CRTC2 (Figs. 3A to 3D).

A factor binding to synoviolin was searched. Since synoviolin has been known as an E3 ubiquitination enzyme (Amano T. et al., Genes Dev 17: 2436-2449), it was verified whether CRTC2 served as a substrate of synoviolin as the E3 ubiquitination enzyme or not. Fig. 4A illustrates the result of the in vitro ubiquitination assay using GST-CRTC2, HA-UA, E1, HbcH5c, and SYVN1ΔTM-His. Consequently, since polyubiquitinated CRTC2 was detected under a condition where all of ATP, HA-UA, E1, HbcH5c, SYVN1ΔTM, and GST-CRTC2 were present, it was confirmed that CRTC2 served as the substrate of synoviolin. Furthermore, siSyvn1 was introduced into INS cells and the CRTC2 expression level was examined, and consequently, an apparent increase of the amount of CRTC2 protein was recognized in the siSyvn1 treatment group compared with the siControl (Figs. 4D, 4E, and 4G).

Next, a transactivation ability of CRTC2 in a synoviolin overexpression system was examined. Fig. 5 illustrates the result. Suppression of the transactivation by the overexpression of synoviolin was recognized for FSK-induced transactivation using HEK293 cells (Fig. 5B), and the suppression of FSK-stimulated transactivation by synoviolin was recognized to be recovered in accordance with the increase of CRTC2 amount (Fig. 5C).

Furthermore, siSyvn1 was introduced into INS cells and the influence on the FSK-stimulated transactivation was examined, and consequently, an apparent increase of the PGC1α expression level was recognized in the siSyvn1 treatment group compared with the siControl (Fig. 5D).

As a result of examining the ATP amount, in a ground state (glucose 3 mM) and at the glucose stimulation (glucose 25 mM), a significant decrease of the ATP amount was recognized in the KO mouse compared with the control mouse (Fig. 5E).

Consequently, it was shown that CRTC2 was the substrate of synoviolin and synoviolin related to the negative control of CRTC2, and it was suggested that the abnormal blood glucose level in the KO mouse was a result of the decrease of the amount of insulin secretion accompanied by the increase of PGC1α and the decrease of the ATP amount due to the CRTC2 overexpression caused by the synoviolin decrease.

### Therapy Experiment Using Animal Model

### Testing Method

For the experiment, under the above-described environment, an action of a test article relative to a change over time of the amount of glucose reactive insulin secretion was examined using a mouse in which Syvn1 of the pancreatic β cells were specifically knocked out and a wild type mouse in the same strain. As the test article, ciclosporin A (CsA) was used. CsA was purchased from Nacalai Tesque (Kyoto, Japan). CsA dissolved in an olive oil (Sigma, St. Louis, MO, US) was administered to the mouse at a dose of 10 mg/kg/day for three weeks. The olive oil was administered to the control mouse. On the last day of the oral administration, glucose was orally administered to the KO and the WT after one night fasting, blood samples were successively obtained 0, 15, 30, 60, and 120 minutes later, and the blood glucose levels were measured by the above-described method.

### Results

Fig. 6B illustrates the changes of blood glucose level when ciclosporin was used as a test compound. As a result, statistically significant decrease of blood glucose level was recognized in the group in which ciclosporin was administered compared with the olive oil (control group). Furthermore, the amount of insulin secretion was increased by the ciclosporin treatment (Fig. 6C). Furthermore, the amounts of glucose responsive insulin secretion were examined using the pancreatic islets of the WT and the KO mice. From the above-described results, for ciclosporin, the statistically significant increase of the amount of insulin secretion by ciclosporin was recognized in the pancreatic islets of the KO mouse (Fig. 6D).

It was confirmed that the nerve specific Syvn1 deficient mouse exhibited a phenotype of convulsion, gait disorder, or the like and died in the first few weeks of life, and since the neurological disorder was supposed to have a relation with WFS1 and Syvn1, the mouse was used as a model mouse of the neurological symptom. A nerve specific Syvn1 hetero deficient mouse (Syn-Cre, Syvn1^{fl/+}) was crossed with a Syvn1 flox mouse (Syvn1^{fl/fl}), thereby producing a nerve specific Syvn1 deficient mouse (Syn-Cre, Syvn1^{fl/fl}). From the 15th day after fertilization to weanling, 0.1 mg/kg of FK506-hydrate, FUJIFILM Wako Pure Chemical Corporation (Osaka City, Japan) dissolved in a phosphate buffered saline was orally administered to the mother body, and the influence on the phenotype of the offspring was examined.

The survival rate of the nerve specific Syvn1 deficient mouse was examined. Fig. 7 illustrates the result. As illustrated in Fig. 7, while the average number of days of living of the mice born from untreated mother bodies was 18.5 days (standard deviation 2.6 days), the average number of days of living of the mice born from the mother bodies to which tacrolimus was administered was 34.7 days (standard deviation 7.6 days). Accordingly, it was suggested that tacrolimus was effective to the neurological disorder mediated by synoviolin.

### Preparation of Reagent and Test Compound Solution

CsA, Nacalai Tesque (Kyoto, Japan), was prepared such that FK506-hydrate, FUJIFILM Wako Pure Chemical Corporation (Osaka City, Japan) medicine was appropriately weighed and dissolved in dimethyl suloxide (DMSO), and then, a culture fluid was used to obtain a final concentration.

### Measurement of Transactivation

The luciferase assay was performed according to a usual method (Chakravart et al., Nature 383: 99-103 (1998)). Briefly describing, seeding was performed on a plate so as to have 1×10⁶ pieces of HEK293 cells, and a transfection was transiently performed with 100 ng of Som-luc reporter plasmid, 25 ng of pcDNA3 HA-CRTC2, 0.01 ng of pRL-SV40, and 20 nM of Syvn1 siRNA. On the other hand, for the SYVN1 overexpression, 100 ng of Som-luc reporter plasmid, 10 ng of pcDNA3 HA-CRTC2, 0.01 ng of pRL-SV40, and 50 or 100 ng of SYVN1 expression vector were transfected. After 24 hours, the cells were treated with 10 µM of FSK for six hours and dissolved in a cell lysis buffer, and then, the luciferase activity was measured by a luminometer (CentroXS3 LB-960 by Berthold) using a commercially available Luc activation measurement kit (Promega). The experiments were each performed at least three times. A suppression rate of the transactivation was calculated by a formula below. Suppression rate (%) = (1 - Luc activity value with test compound and FSK stimulation/Luc activity value with DMSO and FSK stimulation) × 100 (%) For the statistical analysis, one-way analysis of variance was used to determine the correlation. Thereafter, Student's t-test or a multiple comparison analysis was used to analyze an average difference of the luciferase activity between the control mouse and the KO mouse. A P value of less than 0.05 was assumed as a statistically significant difference.

### Result

Fig. 6A illustrates the result. The Luc value indicates the average (number of examples 3). As a result, the CRTC2 transactivation was increased by the FSK stimulation to the siSyvn1introduced cell. To the increase, the treatment of 25 nM of ciclosporin as a calcineurin inhibitor indicated a statistically significant suppressive action.

### Transcriptional Repression Effect (2)

### Preparation and Testing Method of Other Test Article Solution

Furthermore, for tacrolimus (FK506) as the other calcineurin inhibitor, a test compound solution was similarly prepared, and somatostatin transcription was measured by the luciferase measuring procedure similarly to the above.

### Result and Consideration

Table indicates the result of the action of tacrolimus (FK506) as the other calcineurin inhibitor. The Luc value indicates the average (number of examples 4). As a result, the somatostatin transactivation was increased by the FSK stimulation to the siSyvn1 introduced cell. To the increase, tacrolimus as a calcineurin inhibitor indicated a suppressive action.

Table 1 Action of Tacrolimus to CRTC2 Transactivation

**[Table 1]**

| MEDICAL AGENT | siRNA | | | CONCENTRATION | WITHOUT STIMULATION | FSK STIMULATED | SUPPRESSION RATE (%) |
|---|---|---|---|---|---|---|---|
| DMSO | | siControl | | - | 0.13±0.02 | 0.93±0.18 | - |
| DMSO | | | siSyvn1 | 25 ng | 0.16±0.05 | 1.49±0.32 | 0 |
| TACROLIMUS | | siControl | | - | 0.12±0.02 | 0.94±0.29 | - |
| TACROLIMUS | | | siSyvn1 | 25 ng | 0.11±0.03 | 1.25±0.13 | 16.5% |

| | | | | | | | |
|---|---|---|---|---|---|---|---|
| Values of average value ± standard deviation are indicated. | | | | | | | |

From the results described above, since the calcineurin inhibitor of the present invention inhibits the nuclear translocation of CRTC2 due to the synoviolin decrease to increase the insulin secretion, and finally suppresses the increase of the blood glucose level, it was suggested that the calcineurin inhibitor of the present invention was effective for prevention or as a therapeutic agent for the disease of Wolfram syndrome.

For rapamycin as a calcineurin inhibitor, a test compound solution was similarly prepared, and somatostatin transcription was measured by the luciferase measuring procedure similarly to the above. Rapamycin was purchased from FUJIFILM Wako Pure Chemical Corporation (Osaka City, Japan), and the transactivation was measured using rapamycin instead of FK506-hydrate. Table 2 indicates the result.

**[Table 2]**

| MEDICAL AGENT | siRNA | | | CONCENTRATION | WITHOUT STIMULATION | FSK STIMULATED | SUPPRESSION RATE (%) |
|---|---|---|---|---|---|---|---|
| DMSO | | siControl | | - | 0.26±0.05 | 2.07±0.09 | - |
| DMSO | | | siSyvn1 | 25 ng | 0.30±0.04 | 3.10±0.38 | 0 |
| RAPAMYCIN | | siControl | | - | 0.26±0.04 | 1.77±0.25 | - |
| RAPAMYCIN | | | siSyvn1 | 25 ng | 0.28±0.03 | 2.11±0.14 | 32.8% |

As a result, the somatostatin transactivation was increased by the FSK stimulation to the siSyvn1 introduced cell. To the increase, rapamycin as a calcineurin inhibitor indicated a suppressive action.

In the examination of the above-described examples, since it was indicated that cyclosporin, tacrolimus, and rapamycin representative as the calcineurin inhibitor were effective for the therapy of Wolfram syndrome, in consideration of a mechanism of action, it is considered that the calcineurin inhibitor is generally effective to the therapy of Wolfram syndrome.

### INDUSTRIAL APPLICABILITY

The present invention is applicable in the pharmaceutical industry.

## Claims

1. A therapeutic agent for use in the treatment of Wolfram syndrome containing a calcineurin inhibitor as an active ingredient, **characterised in that**
the calcineurin inhibitor is tacrolimus, a pharmaceutically acceptable salt of tacrolimus, or a pharmaceutically acceptable solvate of tacrolimus.

## Patentansprüche

1. Therapeutisches Mittel zur Verwendung bei der Behandlung des Wolfram-Syndroms, das einen Calcineurin-Inhibitor als aktiven Bestandteil enthält, **dadurch gekennzeichnet, dass**
der Calcineurin-Inhibitor Tacrolimus, ein pharmazeutisch akzeptables Salz von Tacrolimus oder ein pharmazeutisch akzeptables Solvat von Tacrolimus ist.

## Revendications

1. Agent thérapeutique utilisé dans le traitement du syndrome de Wolfram, contenant une calcineurine comme principe actif,
caractérisé en ce fait que
l'inhibiteur de la calcineurine est le tacrolimus, un sel de tracrolimus pharmaceutiquement acceptable ou un solvant pharmaceutiquement acceptable du tacrolimus.
